# EUROPEAN PATENT SPECIFICATION

(11) **EP 4 588 462 B1**
(45) Date of publication and mention of the grant of the patent: **05.08.2026**
(21) Application number: 24194023.8
(22) Date of filing: 12.08.2024
(51) Int. Cl.: A61F 13/02, A61B 17/08

(54) **SKIN TENSION-REDUCING SCAR-PREVENTING PLASTER**
HAUTSPANNUNGSREDUZIERENDES NARBENVERHINDERNDES PFLASTER
PANSEMENT POUR LA PRÉVENTION DE CICATRICE RÉDUISANT LA TENSION DE LA PEAU

(30) Priority: 16.01.2024 CN 202420104648 U
(43) Date of publication of application: 23.07.2025
(73) Proprietor: Beijing Easeng Medical Science Co., Ltd., Beijing 100000 (CN)
(72) Inventor: Wang, Wenya, Beijing, 100000 (CN); Peng, Cheng, Beijing, 100000 (CN); Zhao, Xiang, Beijing, 100000 (CN); You, Chaoqun, Beijing, 100000 (CN); Cheng, Linfei, Beijing, 100000 (CN); Lu, Peng, Beijing, 100000 (CN); Gao, Pan, Beijing, 100000 (CN); Yang, Long, Beijing, 100000 (CN)
(74) Representative: Vesterinen, Jussi Tapio

(56) References cited:
- CN-A- 114 617 601
- CN-U- 218 899 555
- US-A1- 2019 046 195
- US-B2- 11 033 270
- US-B2- 9 554 799

## Description

### TECHNICAL FIELD

The present disclosure belongs to the surgical technical field of closed wound, and particularly relates to a skin tension-reducing scar-preventing plaster.

### BACKGROUND

The scar is the appearance and histopathological change of normal skin tissues caused by various wounds, and an inevitable product during human wound repairing. When scar growth exceeds a certain limit, various complications such as appearance destroy and dysfunction will occur. For scar healing and resolution, wound closure and formation of new tissues are mainly from several days to several weeks of the injury in the initial healing stage, and the scar looks reddish, prominent or hard from the initial several weeks to several months after the wound closure; and it usually takes several months to one year even longer time, so the scar will become flat and soft gradually, the color will also fade gradually and be closer to the surrounding normal skin.

In the prior art, the patent No. CN213252506U discloses a novel external scar-removing plaster, including a plaster body, the plaster body includes an outer protective layer, an itching relieving layer, a treatment layer and an isolation box from outside to inside, and the outer protective layer, the itching relieving layer and the treatment layer are fixedly connected in respective; and the bottom of the treatment layer and the upper surface of the isolation box are adhered together through the stickiness of the treatment layer, a partition plate is fixedly connected into the isolation box and divides the isolation box into a first placement cavity and a second placement cavity. US2019046195 discloses another scar reducing device.

It can be known from the prior art that preventing the generation of the skin scar is mainly based on two treatment ideas, firstly, the skin stretching in the scar area is reduced to inhibit the scar, as shown in the above-mentioned disclosed document, and secondly, the scar is reduced through the physical action of a silicone gel, its principle is that the structure of the scar tissue is changed by hydration and closure to the skin stratum corneum, thus allowing the scar to be softened and reduced and preventing the formation of new scar tissues; and the two treatment ideas select to use the corresponding treatment plan according to patient's needs and actual situations.

### SUMMARY

To address the technical issue proposed in the background art, the present disclosure provides a skin tension-reducing scar-preventing plaster as recited in claim 1.

Preferably, a section of surfaces of the traction strips that are close to the fixture blocks are provided with clamping slots, clamping sheets are provided in the fixture blocks, the traction strips pass through the inside of the fixture blocks, and the clamping slots are clamped with the clamping sheets.

Preferably, the clamping slots are composed of a plurality of annular grooves with openings facing the fixing blocks, mounting openings are provided on inner walls of the fixture blocks, one ends of the clamping sheets that face the fixing blocks are fixedly mounted on inner walls of the mounting openings, and the clamping slots are clamped with the clamping sheets through the annular grooves.

Preferably, upper and lower surfaces of the blank area on the film are smooth.

Preferably, the surface of the film is provided with release paper.

Compared with the prior art, the present disclosure has the following beneficial effects: in the present disclosure, the left part is close to the right part through traction, the film in the blank area shrinks first due to a relatively low physical strength, then the skin in the blank area is driven to shrink to the silicone part, such that the tension of the skin tissue in the scope of the silicone part is effectively reduced, thus playing a role in skin tension-reducing, helping the growth and healing of the skin tissue and further reducing the formation of the scar; and the cooperative use of the silicone and the tension-reducing structure plays a role in preventing the scar.

### BRIEF DESCRIPTION OF THE DRAWINGS

FIG. 1 is a schematic diagram of a solid structure of a skin tension-reducing scar-preventing plaster according to the present disclosure.
FIG. 2 is a schematic diagram of a plane structure of a skin tension-reducing scar-preventing plaster according to the present disclosure.
FIG. 3 is a schematic diagram of a front structure of a fixture block.
FIG. 4 is a schematic diagram of a side structure of a fixture block.

In the drawings: 1. Silicone part, 2. Film, 3. Left part, 4. Traction strip, 5. Clamping slot, 6. Fixing block, 7. Fixture block, 8. Mounting opening, 9.Pull ring, 10. Clamping sheet, 11. Blank area, 12. Right part.

### DETAILED DESCRIPTION OF THE EMBODIMENTS

The technical solutions in the embodiments of the present disclosure are clearly and completely elaborated below in combination with the drawings. It is apparent that the described embodiments are only a part of the embodiments of the present disclosure but not all.

In the description of the present disclosure, it is understood that orientation or position relationships indicated by the terms "upper", "lower", "front", "rear", "left", "right", "top", "bottom", "inner", "outer" and the like are based on the orientation or position relationships as shown in the drawings, for ease of describing the present disclosure and simplifying the description only, rather than indicating or implying that the mentioned apparatus or element necessarily has a particular orientation and must be constructed and operated in the particular orientation. Therefore, these terms should not be understood as limitations to the present disclosure.

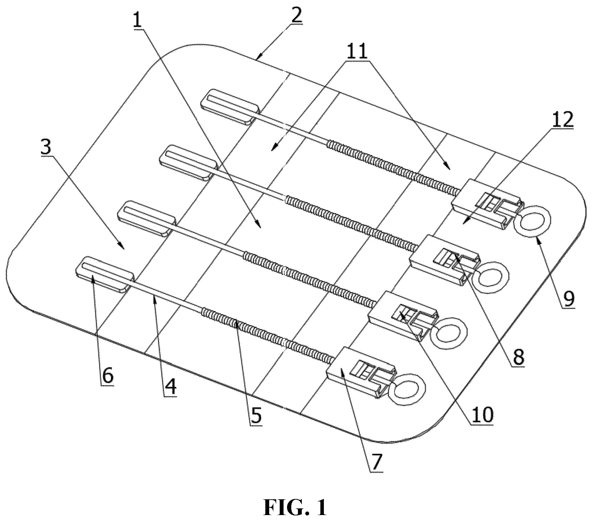

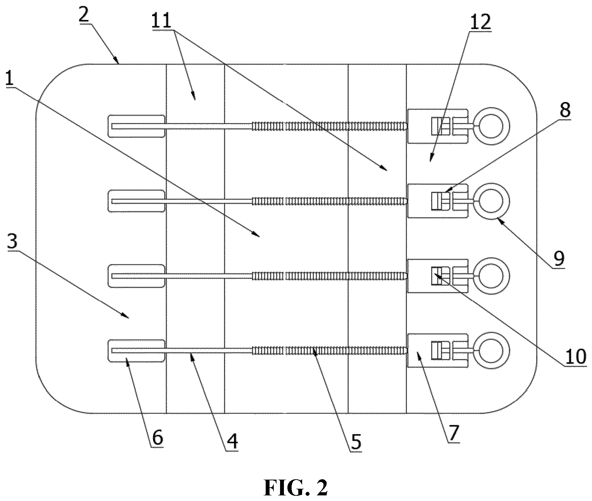

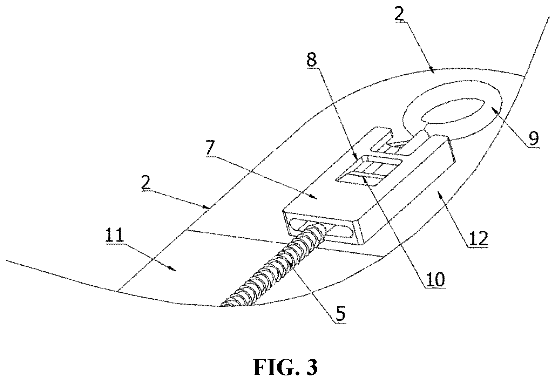

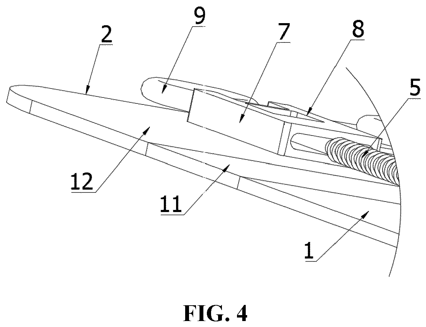

Referring to FIGS. 1-4, a skin tension-reducing scar-preventing plaster, including a film 2 stuck to the skin, where the film 2 is divided into a left part 3, a silicone part 1 and a right part 12, lower surfaces of the left part 3 and the right part 12 are both provided with adhesion gels, a lower surface of the silicone part 1 is provided with a silicone gel, an area between the left part 3 and the silicone part 1 is a blank area 11, and an area between the right part 12 and the silicone part 1 is also a blank area 11; and a plurality of fixing blocks 6 are fixedly mounted on the surface of the left part 3, a plurality of fixture blocks 7 corresponding to the fixing blocks 6 are fixedly mounted on the surface of the right part 12, traction strips 4 are connected between the fixing blocks 6 and the fixture blocks 7, and one ends of the traction strips 4 are fixedly connected with the fixing blocks 6 while the other ends are clamped with the fixture blocks 7.

One ends of the traction strips 4 that are away from the fixing blocks 6 are provided with pull rings 9, the pull rings 9 are used for pulling the traction strips 4, upper and lower surfaces of the blank area 11 on the film 2 are smooth, and the surface of the film 2 is provided with release paper (not shown in the drawings).

A section of surfaces of the traction strips 4 that are close to the fixture blocks 7 are provided with clamping slots 5, clamping sheets 10 are provided in the fixture blocks 7, the traction strips 4 pass through the inside of the fixture blocks 7, and the clamping slots 5 are clamped with the clamping sheets 10. When in use, users pull the traction strips 4 to be close to one ends of the fixture blocks 7, with the pulling, the clamping sheets 10 may be clamped at different positions of the clamping slots 5, thus narrowing the spacing between a second connecting sheet 12 and a first connecting sheet 3, allowing the skin to be reduced in tension and helping the skin recovery. The clamping slots 5 are composed of a plurality of annular grooves with openings facing the fixing blocks 6, mounting openings 8 are provided on inner walls of the fixture blocks 7, one ends of the clamping sheets 10 that face the fixing blocks 6 are fixedly mounted on inner walls of the mounting openings 8, the clamping slots 5 are clamped with the clamping sheets 10 through the annular grooves, and the openings of the annular grooves are arranged in opposite to the clamping sheets 10. In a process of pulling the traction strips 4, the elasticity of the skin tissue has a traction acting force on the fixing blocks 6, meanwhile the elasticity of the tissue also has the traction acting force on the fixture blocks 7, and the acting force on the fixing blocks 6 is opposite to that on the fixture blocks 7, just allowing the annular grooves to be clamped with the clamping sheets 10, thus implementing the fixing effect.

The silicone gel is the existing material, the silicone part 1 can form a layer of film on the wound to lock the moisture on the skin surface, this moisturizing environment helps to reduce the generation of the scar tissue, dryness may promote the formation of the scar tissue, and the silicone part 1 may maintain the temperature and pressure balance in the wound area, to help to reduce the growth of the scar tissue. Through the change of physical properties, the silicone plaster can reduce the hyperplasia of the scar tissue, thus reducing the tension in the scar area to some extent and reducing the possibility of stimulating the scar hyperplasia; the use of the silicone part 1 may reduce the redness and itching feeling of the scar, thus improving the appearance and feeling of the scar; and the silicone part 1 helps to adjust the arrangement of collagen fibers, making the scar smoother and softer.

When in use, the release paper on the lower surface of the film 2 is torn off, the release paper is used for keeping the viscosity of the gel, and the lower surface of the film 2 has the viscosity; first the silicone part 1 is pasted to the patient's scar, the scar-preventing plaster cannot be applied on the opening wound, the users pull the traction strips 4 by the pull rings 9 from one side of the second connecting sheet 12, with the pulling of the traction strips 4, the clamping sheets 10 may be clamped at different positions of the clamping slots 5, pulling will enable the first connecting sheet 3 to close to the second connecting sheet 12, and the tension of the skin tissue in the space position and the scope of the middle sheet 1 will be reduced effectively. In the pulling process, the using surface of the blank area 11 is compounded with nothing, so it is the softest part of the whole plaster and will shrink preferentially, the blank area 11 shows a shrinking state and is not pasted with the skin, so the skin will not shrink with the shrinking of the blank area, and the skin in the blank area will present a tension-reducing effect. In a process of skin healing, the two ends of the unhealed wound have no outward tension, so its new tissues may be pasted tightly for healing, allowing the deep layer of the skin at this part to be consistent with the surface layer in tension, helping the growing and healing of the skin tissue and further preventing the formation of the scar; and the cooperative use of the silicone and the tension-reducing structure plays a role in preventing the scar.

Reducing the skin tension vertical to the wound and away from the wound direction helps to relieve or prevent the formation of the scar, pressure reduction means that reducing the pressure at the wound can improve the blood circulation in this area when preventing the formation of the scar, and the pressure reduction can reduce the inflammatory reaction at the wound; the inflammation is a part of the normal healing process, but the excessive inflammatory reaction may lead to the hyperplasia of the scar tissue and also helps the collagen fibers to heal in a more nature arrangement way, thus reducing the formation of the hard scar and helping the scar tissue to integrate into the surrounding normal tissue better; and reducing the skin tension can reduce the cell stress level, thus reducing the release of chemical signals capable of promoting the formation of the scar. This device can play a more obvious pressure-reducing effect on a relatively great wound or a great-tension wound, such as the vicinity of a joint, chest/back, belly, hip, thigh, neck, high-pressure wound after cutting the enlarged tumor, etc.

In conclusion, the above are only the specific implementation modes of the present disclosure, but the scope of protection of the present disclosure is not limited to this. Those skilled in the art can make equal replacements or changes according to the technical solution and its concept of the present disclosure in the technical scope of the present disclosure, and those replacements or changes shall be covered by the scope of protection limited by the claims.

## Claims

1. A skin tension-reducing scar-preventing plaster, comprising a film (2) stuck to the skin, wherein the film (2) is divided into a left part (3), a silicone part (1) and a right part (12), lower surfaces of the left part (3) and the right part (12) are both provided with adhesion gels, a lower surface of the silicone part (1) is provided with a silicone gel, an area between the left part (3) and the silicone part (1) is a blank area (11), and an area between the right part (12) and the silicone part (1) is also a blank area; and
a plurality of fixing blocks (6) are fixedly mounted on the surface of the left part (3), a plurality of fixture blocks (7) corresponding to the fixing blocks (6) are fixedly mounted on the surface of the right part (12), traction strips (4) are connected between the fixing blocks (6) and the fixture blocks (7), and one ends of the traction strips (4) are fixedly connected with the fixing blocks (6) while the other ends are clamped with the fixture blocks (7); one ends of the traction strips (4) that are away from the fixing blocks (6) are fixedly connected with pull rings (9), and the pull rings (9) are used for pulling the traction strips (4).

2. The skin tension-reducing scar-preventing plaster according to claim 1, wherein a section of surfaces of the traction strips (4) that are close to the fixture blocks (7) are provided with clamping slots (5), clamping sheets (10) are provided in the fixture blocks (7), the traction strips (4) pass through the inside of the fixture blocks (7), and the clamping slots (5) are clamped with the clamping sheets (10).

3. The skin tension-reducing scar-preventing plaster according to claim 2, wherein the clamping slots (5) are composed of a plurality of annular grooves with openings facing the fixing blocks (6), mounting openings (8) are provided on inner walls of the fixture blocks (7), one ends of the clamping sheets (10) that face the fixing blocks (6) are fixedly mounted on inner walls of the mounting openings (8), and the clamping slots (5) are clamped with the clamping sheets (10) through the annular grooves.

4. The skin tension-reducing scar-preventing plaster according to claim 1, wherein upper and lower surfaces of the blank area (11) on the film (2) are smooth.

5. The skin tension-reducing scar-preventing plaster according to claim 1, wherein the surface of the film (2) is provided with release paper.

## Patentansprüche

1. Hautspannungsreduzierendes und narbenverhinderndes Pflaster, umfassend eine auf die Haut aufklebende Folie (2), wobei die Folie (2) in einen linken Teil (3), einen Silikonteil (1) und einen rechten Teil (12) unterteilt ist, wobei untere Oberflächen des linken Teils (3) und des rechten Teils (12) jeweils mit einem Haftgel versehen sind, wobei eine untere Oberfläche des Silikonteils (1) mit einem Silikongel versehen ist, wobei ein Bereich zwischen dem linken Teil (3) und dem Silikonteil (1) ein Leerbereich (11) ist und ein Bereich zwischen dem rechten Teil (12) und dem Silikonteil (1) ebenfalls ein Leerbereich ist;
und wobei mehrere Befestigungsblöcke (6) fest auf der Oberfläche des linken Teils (3) montiert sind, mehrere den Befestigungsblöcken (6) entsprechende Klemmblöcke (7) fest auf der Oberfläche des rechten Teils (12) montiert sind, Zugstreifen (4) zwischen den Befestigungsblöcken (6) und den Klemmblöcken (7) verbunden sind, und jeweils ein Ende der Zugstreifen (4) fest mit den Befestigungsblöcken (6) verbunden ist, während das jeweils andere Ende mit den Klemmblöcken (7) verklemmt ist; die Enden der Zugstreifen (4), die von den Befestigungsblöcken (6) abgewandt sind, sind fest mit Zugringen (9) verbunden, und die Zugringe (9) dienen zum Ziehen der Zugstreifen (4).

2. Hautspannungsreduzierendes und narbenverhinderndes Pflaster nach Anspruch 1, wobei ein den Klemmblöcken (7) benachbarter Oberflächenabschnitt der Zugstreifen (4) mit Klemmnuten (5) versehen ist, Klemmplatten (10) in den Klemmblöcken (7) vorgesehen sind, die Zugstreifen (4) durch das Innere der Klemmblöcke (7) hindurchgeführt sind und die Klemmnuten (5) mit den Klemmplatten (10) verklemmt sind.

3. Hautspannungsreduzierendes und narbenverhinderndes Pflaster nach Anspruch 2, wobei die Klemmnuten (5) aus mehreren ringförmigen Nuten zusammengesetzt sind, deren Öffnungen den Befestigungsblöcken (6) zugewandt sind, Montageöffnungen (8) an Innenwänden der Klemmblöcke (7) vorgesehen sind, jeweils ein den Befestigungsblöcken (6) zugewandtes Ende der Klemmplatten (10) fest an Innenwänden der Montageöffnungen (8) montiert ist und die Klemmnuten (5) durch die ringförmigen Nuten mit den Klemmplatten (10) verklemmt sind.

4. Hautspannungsreduzierendes und narbenverhinderndes Pflaster nach Anspruch 1, wobei die obere und die untere Oberfläche des Leerbereichs (11) auf der Folie (2) glatt sind.

5. Hautspannungsreduzierendes und narbenverhinderndes Pflaster nach Anspruch 1, wobei die Oberfläche der Folie (2) mit einem Trennpapier versehen ist.

## Revendications

1. Patch pour prévention de la cicatrice et réduction de la tension cutanée, comprenant un film (2) à coller à la peau, dans lequel le film (2) est divisé en une partie gauche (3), une partie en silicone (1) et une partie droite (12), des surfaces inférieures de la partie gauche (3) et de la partie droite (12) sont toutes deux pourvues de gels adhésifs, une surface inférieure de la partie en silicone (1) est pourvue d'un gel de silicone, une zone entre la partie gauche (3) et la partie en silicone (1) est une zone blanc (11), et une zone entre la partie droite (12) et la partie en silicone (1) est aussi une zone blanc ; et
une pluralité de blocs de fixation (6) sont montés de manière fixe sur la surface de la partie gauche (3), une pluralité de blocs de positionnement (7) correspondant aux blocs de fixation (6) sont montés de manière fixe sur la surface de la partie droite (12), des bandes de traction (4) sont reliées entre les blocs de fixation (6) et les blocs de positionnement (7), et une partie des extrémités des bandes de traction (4) sont reliées de manière fixe aux blocs de fixation (6) tandis que les autres extrémités sont serrées avec les blocs de positionnement (7) ; les extrémités des bandes de traction (4) qui s'éloignent des blocs de fixation (6) sont reliées de manière fixe à des anneaux de traction (9), et les anneaux de traction (9) sont utilisés pour tirer les bandes de traction (4).

2. Patch pour prévention de la cicatrice et réduction de la tension cutanée selon la revendication 1, dans lequel une section des surfaces des bandes de traction (4) qui sont proches des blocs de positionnement (7) est pourvue de fentes de serrage (5), des plaques de serrage (10) sont prévues dans les blocs de positionnement (7), les bandes de traction (4) passent par l'intérieur des blocs de positionnement (7), et les fentes de serrage (5) sont serrées avec les plaques de serrage (10).

3. Patch pour prévention de la cicatrice et réduction de la tension cutanée selon la revendication 2, dans lequel les fentes de serrage (5) sont composées d'une pluralité de rainures annulaires avec des ouvertures faisant face aux les blocs de fixation (6), des ouvertures de montage (8) sont prévues sur des parois internes des blocs de positionnement (7), unes extrémités des plaques de serrage (10) qui font face aux blocs de fixation (6) sont montées de manière fixée sur les parois internes des ouvertures de montage (8), et les fentes de serrage (5) sont serrées avec les plaques de serrage (10) par les rainures annulaires.

4. Patch pour prévention de la cicatrice et réduction de la tension cutanée selon la revendication 1, dans lequel des surfaces supérieure et inférieure de la zone blanc (11) sur le film (2) sont lisses.

5. Patch pour prévention de la cicatrice et réduction de la tension cutanée selon la revendication 1, dans lequel la surface du film (2) est pourvue d'un papier de protection.
